# EUROPÄISCHE PATENTANMELDUNG

(11) **EP 0 797 989 A1**
(43) Veröffentlichungstag der Anmeldung: **01.10.1997**
(21) Anmeldenummer: 97104344.3
(22) Anmeldetag: 14.03.1997
(51) Int. Cl.: A61K 9/20, A61K 31/42

(54) **Feste Arzneistoffzubereitung, enthaltend leflunomid**

(30) Priorität: 27.03.1996 DE 19612131
(71) Anmelder: HOECHST AKTIENGESELLSCHAFT, 65929 Frankfurt am Main (DE)
(72) Erfinder: Siefke, Verena, Dr., 65929 Frankfurt (DE); Mentrup, Edgar, Dr., 60437 Frankfurt (DE)

(57) **Zusammenfassung**

Eine feste Arzneistoffzubereitung, enthaltend 5-Methylisoxazol-4-carbonsäure-(4-trifluormethyl)-anilid, wird im wesentlichen wasserfrei hergestellt.

## Beschreibung

Aus der Europäischen Patentanmeldung, Veröffentlichungsnummer 0 013 376 A2, ist bekannt, daß 5-Methylisoxazol-4-carbonsäure-(4-trifluormethyl)-anilid (Verbindung 1) antirheumatisch, antiphlogistisch, antipyretisch und analgetisch wirksam ist und gegen multiple Sklerose eingesetzt werden kann. Arzneimittel, enthaltend den Wirkstoff 5-Methylisoxazol-4-carbonsäure-(4-trifluormethyl)-anilid werden in Dosen von 5 mg bis 150 mg oral appliziert.

Aus der Europäischen Patentanmeldung, Veröffentlichungsnummer 0 217 206 A2, ist bekannt, daß N-(4-Trifluormethylphenyl)-2-cyan-3-hydroxy-crotonsäureamid (Verbindung 2) immunmodulierende Eigenschaften hat und sich als Arzneimittel gegen chronische Graft-versus-Host-Krankheiten sowie gegen Autoimmunerkrankungen, insbesondere systemischen Lupus erythematodes, eignet. Arzneimittel, enthaltend den Wirkstoff N-(4-Trifluormethylphenyl)-2-cyan-3-hydroxy-crotonsäureamid, werden in Dosen von 50 mg bis 200 mg appliziert.

Es wurde nun gefunden, daß die Herstellung von festen Arzneistoffzubereitungen, enthaltend Verbindung 1, beispielsweise in Tablettenform, während der Lagerung zur Bildung von 6 % bis 9 % Verbindung 2 führt, wobei die Prozentangabe sich auf Verbindung 1 bezieht. Verbindung 2 wird dabei während der Lagerung aus Verbindung 1 gebildet. Arzneistoffzubereitungen, enthaltend 6 bis 9 % Verunreinigungen durch andere Wirkstoffe genügen einer modernen Therapie nicht, da sie eine genau dosierte, konstante, kontrollierte Applikation der Verbindung 1 erschweren.

Die Erfindung bezweckt durch Modifizierung der Herstellverfahren eine Arzneistoffzubereitung, enthaltend Verbindung 1, zur Verfügung zu stellen, in der wesentlich weniger Verbindung 2, während der Lagerung gebildet wird.

Die Aufgabe wird dadurch gelöst, daß die Herstellung der Arzneistoffzubereitung, enthaltend Verbindung 1, im wesentlichen wasserfrei durchgeführt wird.

Die Erfindung betrifft daher eine feste Arzneistoffzubereitung, enthaltend 5-Methylisoxazol-4-carbonsäure-(4-trifluormethyl)-anilid und einen pharmazeutisch verträglichen Träger, dadurch gekennzeichnet, daß 5-Methylisoxazol-4-carbonsäure-(4-trifluormethyl)-anilid und der pharmazeutisch verträgliche Träger im wesentlichen wasserfrei zu einer festen Arzneistoffzubereitung verpreßt werden.

Die Herstellung von 5-Methylisoxazol-4-carbonsäure-(4-trifluormethyl)-anilid erfolgt gemäß EP 0 013 376. Als pharmazeutisch verträglicher Träger können beispielsweise Laktose, Maisstärke, Polyvinylpyrrolidon, hochdisperses Siliziumdioxid, Cellulose, Polyethylenglykol, Polyglykol oder Magnesiumstearat eingesetzt werden.

Die erfindungsgemäßen festen Arzneistoffzubereitungen weisen nach 6 Monaten Lagerung bei 40 °C und 75 % Feuchtigkeit in der Luft einen Gehalt an N-(4-Trifluormethylphenyl)-2-cyan-3-hydroxy-crotonsäureamid von 1,0 bis 4,5 %, in der Regel von 1,5 % bis 4 % insbesondere von 1,5 % bis 3,5 % auf; jeweils bezogen auf den Gehalt der Verbindung 1, die als 100 % gesetzt wird.

Der Bergriff "feste Arzneistoffzubereitung" bedeutet Zubereitungen wie Tabletten, Kapsel, Pulver, Suppositorien oder Granulat. Der Begriff "im wesentlichen wasserfrei" bedeutet, daR trockener Wirkstoff und trockene Hilfsstoffe eingesetzt werden und kein zusätzliches Wasser bei der Herstellung der Arzneistoffzubereitung eingesetzt wird.

Der Gehalt von 5-Methylisoxazol-4-carbonsäure-(4-trifluormethyl)-anilid in einer festen Arzneistoffzubereitung beträgt 2 mg bis 250 mg, bevorzugt 5 mg bis 150 mg, insbesondere 10 mg bis 50 mg, insbesondere bevorzugt 10 mg bis 20 mg.

Die Herstellung der erfindungsgemäßen Arzneistoffzubereitung erfolgt beispielsweise durch Direkttablettierung, Trockengranulation und Tablettierung oder durch Herstellung von Schmelzerstarrungsgranulation und anschließender Tablettierung. Bei der Direkttablettierung wird die Bindung zwischen den Pulverpartikeln durch die Anwendung von hohen mechanischen Drücken bewirkt (Pharmazeutische Technologie, Bauer, Frömming, Führer, 3. Auflage, Georg Thieme Verlag Stuttgart, New York (1991) Seiten 292 - 307). Die Schmelzerstarrungsgranulate werden entweder durch Schmelzen und Schockerstarren, durch Ausgießen und Zerkleinern oder durch Sprüherstarren in Sprühtürmen hergestellt (Pharmazeutische Technologie, Seite 295). Bei der Trockengranulation werden die Bindungen zwischen den zu granulierenden Pulverpartikeln durch die Anwendung von hohen mechanischen Drücken bewirkt. Dies kann sowohl mit Tablettenpressen erreicht werden, wobei als Zwischenprodukte größere Tabletten oder Briketts entstehen, als auch mit Kompaktierwalzen, die Schülpen ergeben. Die erhaltenen Schülpen oder Briketts werden anschließend mit gegenläufigen Stachelwalzen zerkleinert und/oder durch Siebe geschlagen (Pharmazeutische Technologie, Seite 295). Beispielsweise werden die Verbindung 1 und ein oder mehrere der pharmazeutisch verträglichen Trägerstoffe ohne Zusatz von Wasser vermischt. Anschließend werden die trockenen Hilfsstoffe wie Binde-, Fließ-, Gleit- und Schmiermittel zugefügt und zu festen Tabletten verpreßt.

Ferner kann die Verbindung 1 mit Laktose, mikrokristalliner Cellulose und Macrogol 6000 (Polyethylenglykol) in einem schnellaufenden Mischer gemischt werden, wobei sich das Material durch Friktion erwärmt und ein Granulat bildet. Das abgekühlte Granulat wird durch ein Sieb mit 1,2 mm Maschenweite passiert und mit Magnesiumstearat bestäubt. Anschließend wird das Granulat zu Tabletten verpreßt.

Die erfindungsgemäßen festen Arzneistoffzubereitungen unterscheiden sich von Tabletten, die durch Feuchtgranulation erzeugt wurden, durch ein unterschiedliches Freisetzungsverhalten des Wirkstoffes. Die erfindungsgemäße festen Arzneistoffzubereitung zeigt eine Freisetzung von 70 % bis 85 % des Wirkstoffes nach 15 Minuten, bevorzugt von 76 % bis 80 % und nach 30 Minuten von 85 % bis 95 %, bevorzugt von 88 % bis 92 %.

Tabletten, die nach der Feuchtgranulationsmethode hergestellt wurden, zeigen eine Freisetzung von 87 % bis 92 % des Wirkstoffs nach 15 Minuten, und nach 30 Minuten eine Freisetzung von 93 % bis 98 %.

### Beispiel 1

### Direkttablettierung

Für eine Tablette werden benötigt:
- 10,0 mg: 5-Methylisoxazol-4-carbonsäure-(4-trifluormethyl)-anilid (I)
- 78,0 mg: Laktose (II); (Meggle Milchindustrie GmbH & Co. KG, Wasserburg)
- 50,0 mg: Maisstärke (III); (Cerestar Benelux B. V., Sas van Gent, Niederlande)
- 0,5 mg: hochdisperses Siliziumdioxid (IV); (Degussa AG, Rheinfelden)
- 7,5 mg: quervernetztes Poly(1-vinyl-2-pyrrolidon) (V); (BASF, Ludwigshafen) und
- 0,5 mg: Magnesiumstearat (VI); (Otto Bälocher GmbH, München)

Die Komponenten I bis III werden in einem Schaufelmischer 5 Minuten gemischt. Anschließend werden die Komponenten IV bis VI zugefügt und weitere 60 Sekunden gemischt (preßfertige Mischung). Die preßfertige Mischung wird zu Tabletten mit einem Endgewicht von je 146,5 mg verpreßt.

Die Tabletten weisen nach 6 Monaten Lagerung, bei 40 °C mit einer Luftfeuchtigkeit von 75 %, einen Gehalt der Verbindung 2 von 1,5 % auf, bezogen auf den Gehalt der Verbindung 1.

### Beispiel 2

### Sprühgranulation

Für 1 Tablette werden benötigt:
- 10,0 mg: 5-Methylisoxazol-4-carbonsäure-(4-trifluormethyl)-anilid (I)
- 78,0 mg: Laktose (II)
- 50,0 mg: Maisstärke (III)
- 3,5 mg: Poly(1-vinyl-2-pyrrolidon) K25 (IV); (BASF, Ludwigshafen)
- 0,5 mg: hochdisperses Siliziumdioxid (V)
- 7,5 mg: quervernetztes Poly(1-vinyl-2-pyrrolidon) (VI)
- 0,5 mg: Magnesiumstearat (VII)

Die Komponenten I bis III werden in einem Taumelmischer 5 Minuten gemischt. Poly(1-vinyl-2-pyrrolidon) wird in Wasser gelöst (5 bis 7,5 prozentige Lösung) und in einer Sprühapparatur bei einer Zulufttemperatur von etwa 60 °C und einer Ablufttemperatur von etwa 21 °C auf die vermischten Komponenten I bis III aufgesprüht. Anschließend wird das Produkt getrocknet. Das erhaltene Sprühgranulat wird in einen Mischer überführt und mit den Komponenten V bis VII 60 Sekunden gemischt. Die preßfertige Mischung wird wie in Beispiel 1 zu Tabletten mit einem Endgewicht von 150 mg verarbeitet.

Die Tabletten weisen nach 6 Monaten Lagerung, bei 40 °C mit einer Luftfeuchtigkeit von 75 %, einen Gehalt der Verbindung 2 von 8,3 % auf, bezogen auf die Verbindung 1.

### Beispiel 3

### Freisetzung von Verbindung 1 aus Tabletten

Je 1 Tablette gemäß der Beispiele 1 und 2 wird in je 100 ml Wasser gebracht. Nach 15 Minuten und nach 30 Minuten wird die Menge von Verbindung 1 im Überstand bestimmt. Tabelle 1 zeigt die Ergebnisse.

**Tabelle 1**

| Tablette gemäß | Freisetzung nach 15 Minuten | Freisetzung nach 30 Minuten |
|---|---|---|
| Beispiel 2 | 92 % ± 3,1 % | 98 % ± 1,1 % |
| Beispiel 1 | 77 % ± 1,0 % | 88 % ± 1,1 % |

## Patentansprüche

1. Verfahren zur Herstellung einer festen Arzneistoffzubereitung, enthaltend 5-Methylisoxazol-4-carbonsäure-(4-trifluormethyl)-anilid, dadurch gekennzeichnet, daß die Arzneistoffzubereitung im wesentlichen wasserfrei hergestellt wird.

2. Verfahren gemäß Anspruch 1, dadurch gekennzeichnet, daß eine Direkttablettierung oder eine Trockengranulation mit anschließender Tablettierung durchgeführt wird.

3. Verfahren gemäß Anspruch 1, dadurch gekennzeichnet, daß eine Schmelzerstarrungsgranulation durchgeführt wird.

4. Verfahren gemäß einem oder mehreren der Ansprüche 1 bis 3, dadurch gekennzeichnet, daß man als Hilfsstoffe Laktose, Maisstärke, hochdisperses Siliziumdioxid, quervernetztes Poly(1-vinyl-2-pyrrolidon), mikrokristalline Cellulose, Polyethylenglykol, Polyglykol oder Magnesiumstearat einsetzt.

5. Feste Arzneistoffzubereitung, erhältlich durch Direkttablettierung von 5-Methylisoxazol-4-carbonsäure-(4-trifluormethyl)-anilid und einen pharmazeutisch verträglichen Träger.

6. Feste Arzneistoffzubereitung, erhältlich durch Trockengranulation oder Schmelzerstarrungsgranulation und Tablettierung von 5-Methylisoxazol-4-carbonsäure-(4-trifluormethyl)-anilid und einen pharmazeutisch verträglichen Träger.

7. Feste Arzneistoffzubereitung, gemäß der Ansprüche 5 oder 6, dadurch gekennzeichnet, daß man als pharmazeutisch verträglichen Träger Laktose, Maisstärke, hochdisperses Siliziumdioxid, quervernetztes Poly(1-vinyl-2-pyrrolidon), mikrokristalline Cellulose, Polyethylenglykol, Polyglykol oder Magnesiumstearat einsetzt.
